# EUROPÄISCHE PATENTSCHRIFT

(11) **EP 1 523 462 B1**
(45) Veröffentlichungstag und Bekanntmachung des Hinweises auf die Patenterteilung: **17.03.2010**
(21) Anmeldenummer: 03763815.2
(22) Anmeldetag: 10.07.2003
(51) Int. Cl.: C07C 5/333, C07C 11/167

(54) **VERFAHREN ZUR HERSTELLUNG VON BUTADIEN AUS N-BUTAN**
METHOD FOR THE PRODUCTION OF BUTADIENE FROM N-BUTANE
PROCEDE DE PRODUCTION DE BUTADIENE A PARTIR DE N-BUTANE

(30) Priorität: 12.07.2002 DE 10231632
(43) Veröffentlichungstag der Anmeldung: 20.04.2005
(73) Patentinhaber: BASF SE, 67056 Ludwigshafen (DE)
(72) Erfinder: SCHINDLER, Götz-Peter, 68219 Mannheim (DE); WALSDORFF, Christian, 67059 Ludwigshafen (DE); HARTH, Klaus, 67317 Altleiningen (DE); HIBST, Hartmut, 69198 Schriesheim (DE)
(86) Internationale Anmeldenummer: PCT/EP2003/007523
(87) Internationale Veröffentlichungsnummer: WO 2004/007408

(56) Entgegenhaltungen:
- DE-A- 1 543 152
- GB-A- 508 764
- GB-A- 628 686
- GB-A- 1 391 649

## Beschreibung

Butadien wird überwiegend durch thermische Spaltung (Cracken) gesättigter Kohlenwasserstoffe hergestellt, wobei üblicherweise von Naphtha als Rohstoff ausgegangen wird. Beim Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an. Acetylenisch ungesättigte Kohlenwasserstoffe im Crackgas wie Acetylen, Propin, 1-Butin, 2-Butin, Butenin und Diacetylen können z. B. bei einer nachfolgenden Dimerisierung von Butadien in einer Diels-Alder-Reaktion zu Vinylcyclohexen störend sein, da schon Spuren dieser Verbindungen den kupferhaltigen Dimerisierungskatalysator vergiften können. Butine und Allene reagieren ebenfalls in einer Diels-Alder-Reaktion mit Butadien und führen zu Nebenproduktbildung. Auch bei anderen Verwendungen von Butadien sind dreifach ungesättigte C₄-Kohlenwasserstoffe im allgemeinen störend.

Probleme bereiten insbesondere die Butine, die sich von Butadien nur sehr schwer destillativ oder extraktiv abtrennen lassen. Daher ist es bei der Verwendung von Butadien aus Crackern erforderlich, der Butadien-Dimerisierung eine Hydrierstufe vorzuschalten, in der die Butine selektiv zu den entsprechenden Butenen partiell hydriert werden.

Nachteilig ist ferner, dass beim Cracken von Naphtha oder anderen KohlenwasserstoffGemischen ein komplexes Kohlenwasserstoff-Gemisch erzeugt wird. So fallen bei der Erzeugung von Butadien im Crackprozess zwangsläufig größere Mengen an Ethen oder Propen als Koppelprodukte an.

Alternativ kann Butadien ausgehend von n-Butan durch katalytische Dehydrierung hergestellt werden. Nachteilig an diesen Verfahren ist jedoch deren geringe Butadien-Ausbeute, da bei der katalytischen Dehydrierung von n-Butan überwiegend 1-Buten und 2-Buten entstehen.

Aufgabe der Erfindung ist es, ein Verfahren zur Herstellung von Butadien aus n-Butan bereitzustellen, das die Nachteile des Standes der Technik nicht aufweist und mit dem hohe Butadien-Ausbeuten erzielt werden können.

Gelöst wird die Aufgabe durch ein Verfahren zur Herstellung von Butadien aus n-Butan mit den Schritten
(A) Bereitstellung eines n-Butan enthaltenden Einsatzgasstroms,
(B) Einspeisung des n-Butan enthaltenden Einsatzgasstroms in eine erste Dehydrierzone und nicht-oxidative katalytische Dehydrierung von n-Butan zu 1-Buten, 2-Buten und gegebenenfalls Butadien, wobei ein n-Butan, 1-Buten, 2-Buten, gegebenenfalls Butadien und gegebenenfalls Nebenbestandteile enthaltender erster Produktgasstrom erhalten wird,
(C) Einspeisung des n-Butan, 1-Buten, 2-Buten, gegebenenfalls Butadien und gegebenenfalls Nebenbestandteile enthaltenden ersten Produktgasstroms in eine zweite Dehydrierzone und oxidative Dehydrierung von 1-Buten und 2-Buten zu Butadien, wobei ein Butadien, n-Butan, Wasserdampf und gegebenenfalls Nebenbestandteile enthaltender zweiter Produktgasstrom erhalten wird,
(D) Gewinnung von Butadien aus dem zweiten Produktgastrom.

In einem ersten Verfahrensteil A wird ein n-Butan enthaltender Einsatzgasstrom bereitgestellt. Üblicherweise wird dabei von an n-Butan reichen Gasgemischen wie liquefied petroleum gas (LPG) als Rohstoff ausgegangen. LPG enthält im wesentlichen C₂-C₅-Kohlenwasserstoffe. Die Zusammensetzung von LPG kann stark schwanken. Vorteilhafter Weise enthält das eingesetzte LPG mindestens 10 Gew.-% Butane.

In einer Variante des erfindungsgemäßen Verfahrens umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und Pentanen aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und Pentanen erfolgt in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (Pentane) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

In einem Verfahrensteil (B) wird der n-Butan enthaltende Einsatzgasstrom in eine erste Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei gegebenenfalls auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden.

Ein Merkmal der nicht-oxidativen Fahrweise gegenüber einer oxidativen Fahrweise ist das Vorhandensein von Wasserstoff (GB 628 686) im Austragsgas. Bei der oxidativen Dehydrierung wird kein freier Wasserstoff in wesentlichen Mengen gebildet.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan=Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GSHV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne sauerstoffhaltigem Gas als Co-Feed in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysätorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei--mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthält im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispiel 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit Wasserdampf mit einem wasserstoffhaltigen Gas reduziert.

Bei der n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variierten. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.-% 1-Buten, 1 bis 20 Vol.-% 2-Buten, 20 bis 70 Vol.-% n-Butan, 5 bis 70 Vol.-% Wasserdampf, 0 bis 5 Vol.-% leichtsiedene Kohlenwasserstoffe (Methan, Ethan, Ethen, Propan und Propen), 0 bis 30 Vol.-% Wasserstoff, 0 bis 30 Vol.-% Stickstoff und 0 bis 5 Vol.-% Kohlenstoffoxide.

Der nicht-oxidativen katalytischen Dehydrierung wird erfindungsgemäß eine oxidative Dehydrierung (Oxidehydrierung) als Verfahrensteil C nachgeschaltet.

Diese kann grundsätzlich in allen aus dem Stand der Technik bekannten Reaktortypen und Fahrweisen durchgeführt werden, wie beispielsweise im Wirbelbett, im Hordenofen oder im Festbettrohr- oder -rohrbündelreaktor. Letzterer wird bei dem erfindungsgemäßen Verfahren bevorzugt eingesetzt. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der katalytischen Dehydrierung stammende Produktgasgemisch mit Sauerstoff oder einem sauerstoffhaltigem Gas, beispielsweise Luft, vermischt. Das erhaltene sauerstoffhaltige Gasgemisch wird dann der Oxidehydrierung zugeführt.

Für die Oxidehydrierung der n-Butene zu 1,3-Butadien besonders geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ), worauf explizit Bezug genommen wird.

Die Stöchiometrie der Aktivmasse einer Vielzahl der für die Oxidehydrierung der n-Butene zu 1,3-Butadien geeigneten Multimetalloxidkatalysatoren lässt sich unter die allgemeine Formel (I)

Mo₁₂BiₐFe_{b}Co_{c}Ni_{d}CrₑX¹_{f}K_{g}Oₓ (I),

subsumieren in der die Variablen nachfolgende Bedeutung aufweisen:
- X¹ =: W, Sn, Mn, La, Ce, Ge, Ti, Zr, Hf, Nb, P, Si, Sb, Al, Cd und/oder Mg;
- a =: 0,5 bis 5, vorzugsweise 0,5 bis 2;
- b =: 0 bis 5, vorzugsweise 2 bis 4;
- c =: 0 bis 10, vorzugsweise 3 bis 10;
- d =: 0 bis 10;
- e =: 0 bis 10, vorzugsweise 0,1 bis 4;
- f =: 0 bis 5, vorzugsweise 0,1 bis 2;
- g =: 0 bis 2, vorzugsweise 0,01 bis 1; und
- x =: eine Zahl, die durch die Wertigkeit und Häufigkeit der von Sauerstoff verschiedenen Elemente in (I) bestimmt wird.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe_{0,1}Ni₈ZrCr3-K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ). Herstellung und Charakterisierung der genannten Katalysatoren sind ausführlich in den zitierten Schriften beschrieben, auf die hiermit explizit verwiesen wird.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise Tabletten, Zylinder, Hohlzylinder, Ringe, Kugeln, Stränge, Wagenräder oder Extrudate genannt. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als sogenannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung der n-Butene zu Butadien wird im Allgemeinen bei einer Temperatur von 220 bis 490 °C und bevorzugt von 250 bis 450 °C durchgeführt. Aus praktischen Erwägungen wählt man in der Regel einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung an Katalysatoren und inerten Anteilen ab.

Durch die Kopplung der nicht-oxidativen katalytischen, bevorzugt autothermen Dehydrierung mit der oxidativen Dehydrierung der gebildeten n-Butene wird eine sehr viel höhrere Ausbeute an Butadien, bezogen auf eingesetztes n-Butan, erhalten. Ferner kann die nicht-oxidative Dehydrierung schonender betrieben werden. Vergleichbare Ausbeuten wären mit einer ausschließlich nicht-oxidativen Dehydrierung nur zum Preis deutlich niedrigerer Selektivitäten zu erreichen.

Der die oxidative Dehydrierung verlassende zweite Produktgasstrom enthält neben Butadien und nicht umgesetztem n-Butan Wasserdampf. Als Nebenbestandteile enthält er im allgemeinen Kohlenmonoxid, Kohlendioxid, Sauerstoff, Stickstoff, Methan, Ethan, Ethen, Propan und Propen, gegebenenfalls Wasserstoff sowie sauerstoffhaltige Kohlenwasserstoffe, sogenannte Oxygenate. Im Allgemeinen enthält er nur noch geringe Anteile an 1-Buten und 2-Buten.

Beispielsweise kann der die oxidative Dehydrierung verlassende Produktgasstrom 1 bis 20 Vol.-% Butadien, 0 bis 1 Vol.-% 1-Buten, 0 bis 1 Vol.-% 2-Buten, 0 bis 50 Vol.-% Butan, 2 bis 50 Vol.-% Wasserdampf, 0 bis 5 Vol.-% leichtsiedene Kohlenwasserstoffe (Methan, Ethan, Ethen, Propan und Propen), 0 bis 20 Vol.-% Wasserstoff, 0 bis 90 Vol.-% Stickstoff, 0 bis 5 Vol.-% Kohlenstoffoxide und 0 bis 3 Vol.-% Oxygenate enthalten.

Aus dem bei der oxidativen Dehydrierung erhaltenen zweiten Produktgasstrom wird in einem Verfahrensteil D Butadien gewonnen.

Die Gewinnung von Butadien aus dem zweiten Produktgasstrom kann folgende Schritte umfassen:
(D1) Abkühlung - des Produktgasstroms mit Wasser, wobei Wasserdampf und gegebenenfalls hochsiedende organische Nebenbestandteile auskondensiert werden;
(D2) Abtrennung der in dem zweiten Produktgasstrom enthaltenen leichtsiedenden Nebenbestandteile, ausgewählt aus der Gruppe bestehend aus Wasserstoff, Kohlenmonoxid, Kohlendioxid, Stickstoff, Methan, Ethan, Ethen, Propan und Propen, wobei ein Butadien, n-Butan, gegebenenfalls 1-Buten und 2-Buten und gegebenenfalls Oxygenate als weitere Nebenbestandteile enthaltender Strom erhalten wird;
(D3) gegebenenfalls Abtrennung der Oxygenate, wobei ein Butadien, n-Butan und gegebenenfalls 1-Buten und 2-Buten enthaltender Strom erhalten wird;
(D4) Auftrennung des Butadien, n-Butan, gegebenenfalls 1-Buten und 2-Buten enthaltenden Stroms in einen n-Butan und gegebenenfalls 1-Buten und 2-Buten enthaltenden Strom und einen Butadien enthaltenden Strom;
(D5) gegebenenfalls Rückführung des n-Butan und gegebenenfalls 1-Buten und 2-Buten enthaltenden Stroms in die nicht-oxidative katalytische Dehydrierung (B).

Nach Verlassen der Dehydrierstufen wird das heiße Gasgemisch, dessen Temperatur in der Regel von 220 bis 490 °C beträgt, üblicherweise mit Wasser abgekühlt. Dabei werden Wasserdampf und gegebenenfalls hochsiedende organische Nebenbestandteile auskondensiert.

Die in dem Dehydriergasgemisch neben Butadien, n-Butan und gegebenenfalls 1-Buten und 2-Buten enthaltenen leichtsiedenden Nebenbestandteile wie Wasserstoff, Kohlenmonoxid, Kohlendioxid, Stickstoff, Methan, Ethan, Ethen, Propan und Propen werden nachfolgend von den C₄-Kohlenwasserstoffen abgetrennt.

Die Abtrennung der leichtsiedenden Nebenbestandteile kann durch übliche Rektifikation erfolgen.

Die Abtrennung der leichtsiedenden Nebenbestandteile kann auch in einem Absorptions/Desorptions-Cyclus mittels eines hochsiedenden Absorptionsmittels erfolgen. Auf diese Weise werden im wesentlichen alle leichtsiedenden Nebenbestandteile (Stickstoff, Wasserstoff, Methan, Ethan, Ethen, Propan, Propen, Kohlenstoffoxide, Sauerstoff) aus dem Produktgasstrom der n-Butan-Dehydrierung abgetrennt.

Dazu werden in einer Absorptionsstufe die C₄-Kohlenwasserstoffe in einem inerten Absorptionsmittel absorbiert, wobei ein mit den C₄-Kohlenwasserstoffen beladenes Absorptionsmittel und ein die übrigen Nebenbestandteile enthaltendes Abgas erhalten werden. In einer Desorptionsstufe werden die C₄-Kohlenwasserstoff und Spuren von Nebenbestandteilen aus dem Absorptionsmittel wieder freigesetzt.

In der Absorptionsstufe eingesetzte inerte Absorptionsmittel sind im allgemeinen hochsiedende unpolare Lösungsmittel, in denen das abzutrennende Kohlenwasserstoff-Gemisch eine deutlich höhere Löslichkeit als die übrigen Bestandteile der Produktgasmischung aufweisen. Die Absorption kann durch einfaches Durchleiten der Produktgasmischung durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

Es verbleibt ein im wesentlichen aus Butadien und n-Butan bestehender Strom, der noch 1-Buten und 2-Buten sowie so genannte Oxygenate als weitere Nebenbestandteile enthalten kann. Derartige Oxygenate umfassen beispielsweise Furan und Maleinsäureanhydrid. Die Oxygenate können in einer weiteren Trennstufe, die ebenfalls als Absorptions/Desorptionsstufe oder als Rektifikation ausgestaltet sein kann, von den C₄-Kohlenwasserstoffen abgetrennt werden.

Der verbleibende Strom, der üblicher Weise im wesentlichen aus Butadien und n-Butan besteht und daneben noch in geringen Mengen 1-Buten und 2-Buten enthalten kann, kann in einer weiteren Trennstufe in einen n-Butan und gegebenenfalls 1-Buten und 2-Buten enthaltenden Strom und einen Butadien enthaltenden Strom aufgetrennt werden. Die Auftrennung kann beispielsweise durch Butadien-Wäsche erfolgen. Die Butadien-Wäsche kann wie in Weissermehl/Arpe, Industrielle Organische Chemie, 5. Auflage 1998, S. 120/121, oder Hydrocarbon Processing, März 2002, S. 50B beschrieben, erfolgen.

Der n-Butan und gegebenenfalls 1-Buten und 2-Buten enthaltende Strom kann zumindest teilweise in die nicht-oxidative katalytische Dehydrierung (B) zurückgeführt werden.

Vorzugsweise umfasst der Verfahrensteil (D) mindestens die Schritte (D1), (D2) und (D4). Besonders bevorzugt umfasst er die Schritte (D1) bis (D5).

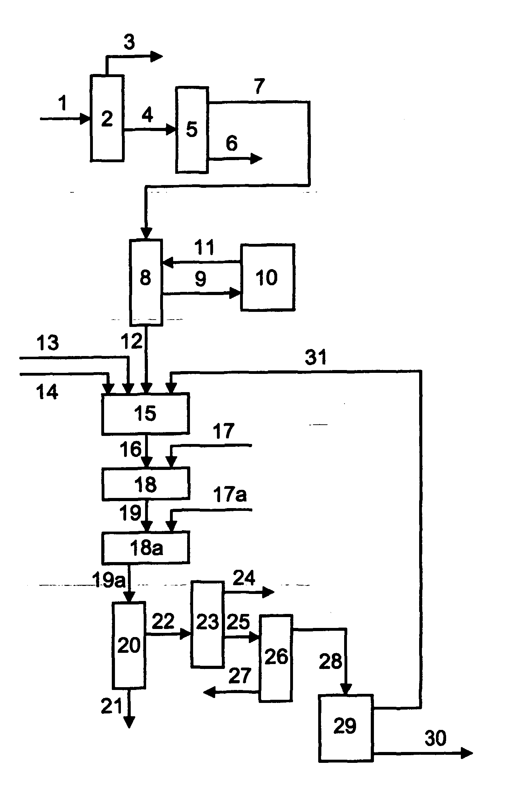

Die Erfindung wird nachfolgend mit Bezugnahme auf die Zeichnung näher erläutert.

Die Figur zeigt das Verfahrensfließbild einer bevorzugten Ausführungsform des erfindungsgemäßen Verfahren. Ein Einsatzstrom 1 aus liquefied petroleum gas (LPG), das im wesentlichen aus Propan, n-Butan und Isobutan besteht und daneben auch Methan, Ethan oder Pentane enthalten kann, wird einer Rektifizierkolonne 2 zugeführt und in einen Strom 3 aus im wesentlichen Propan und gegebenenfalls Methan und Ethan und einen Strom 4 aus im wesentlichen n-Butan und Isobutan und gegebenenfalls Pentanen aufgetrennt. In der Rektifizierkolonne 5 werden gegebenenfalls Pentane 6 abgetrennt. Das Butan-Gemisch 7 wird in der Rektifizierkolonne 8 in Isobutan 9 und n-Butan 12 aufgetrennt, wobei Isobutan in dem Isomerisierungsreaktor 10 zu einem n-Butan/Isobutan-Gemisch 11 isomerisiert wird, das in die Rektifizierkolonne 8 zurückgespeist wird. n-Butan wird als Einsatzgasstrom 12 in die erste Dehydrierstufe 15 eingespeist, in der eine nicht-oxidative katalytische Dehydrierung von Butan zu 1-Buten, 2-Buten und Butadien stattfindet. Diese wird vorzugsweise unter autothermalen Bedingungen unter Zuspeisung von Sauerstoff oder Luft als Co-Feed 13 und optional von Wasserstoff als Co-Feed 14 durchgeführt. Vorzugsweise wird die erste Dehydrierstufe mit Rückvermischung im Wirbelbett oder mit Teilkreisgasführung durchgeführt, beispielsweise wie in der nichtvorveröffentlichten deutschen Patentanmeldung P 102 11 275.4 beschrieben. Der die erste Dehydrierstufe verlassende Produktgasstrom 16, der neben Butadien, 1-Buten, 2-Buten und nicht umgesetztem n-Butan Wasserdampf und üblicherweise Nebenbestandteile wie Wasserstoff, Kohlenstoffoxide, Stickstoff, Wasserstoff, Methan, Ethan, Ethen, Propan und Propen enthält, wird einer zweiten Dehydrierstufe 18 zugeführt, in der unter Zuspeisung von Sauerstoff oder Luft als Co-Feed 17 eine oxidative Dehydrierung von 1-Buten und 2-Buten zu Butadien stattfindet. Die zweite Dehydrierstufe wird vorzugsweise in einem Rohrbündelreaktor durchgeführt. Die zweite Dehydrierstufe kann auch selbst mehrstufig, beispielsweise zweistufig, durchgeführt werden. Bei zweistufiger Ausführung der oxidativen Dehydrierung besteht die zweite Dehydrierstufe aus einer ersten oxidativen Dehydrierstufe 18 und einer zweiten oxidativen Dehydrierstufe 18a, wobei jeweils Luft oder Sauerstoff als Co-Feed 17 bzw. 17a zugespeist wird. Der die zweite Dehydrierstufe verlassende zweite Produktgasstrom 19a (bei einstufiger Ausführung der oxidativen Dehydrierung ist dies der Produktgasstrom 19) enthält neben Butadien und nicht umgesetztem n-Butan Wasserdampf und Nebenbestandteile wie Wasserstoff, Kohlenstoffoxide, Stickstoff, Methan, Ethan, Ethen, Propan und/oder Propen, gegebenenfalls geringe Restmengen 1-Buten und 2-Buten und gegebenenfalls Sauerstoff und sauerstoffhaltige Kohlenwasserstoffe (Oxygenate). Der Produktgasstrom 19a wird, gegebenenfalls nach Vorkühlung in Wärmetauschern, in der Kühl- und Kondensationseinheit 20, die beispielsweise als Wasserrieselbett oder als Fallfilmkondensator ausgebildet sein kann, soweit abgekühlt, dass Wasser und hochsiedende organische Nebenprodukte wie hochsiedende Kohlenwasserstoffe und Oxygenate auskondensieren, die als Strom 21 aus dem Verfahren ausgeschleust werden. Die nicht auskondensierten Produktgasbestandteile werden als Strom 22 der Trennstufe 23 zugeführt, in der eine Abtrennung von Leichtsiedern und nicht kondensierbaren Nebenbestandteilen 24 (sofern im Produktgasstrom 19a vorhanden: Wasserstoff, Kohlenstoffoxide, Stickstoff, Methan, Ethan, Ethen, Propan, Propen und Sauerstoff) stattfindet. Die Trennstufe 23 kann als Rektifizierkolonne oder als Absorptions/Desorptionseinheit ausgestaltet sein. Der die C₄-Produkte der Dehydrierung, nicht umgesetztes n-Butan und gegebenenfalls Oxygenate wie Furan und Maleinsäureanhydrid enthaltende Strom 25 wird optional einer weiteren Trennstufe 26 zugeführt, die als Rektifizierkolonne oder Absorptions-/Desorptionseinheit ausgestaltet sein kann. In der Trennstufe 26 erfolgt eine Abtrennung von Oxygenaten und gegebenenfalls von verbliebenen Wasserspuren, die als Strom 27 aus dem Verfahren ausgeschleust werden. Der Strom 28 aus Butadien und n-Butan, der noch geringe Anteile an 1-Buten und 2-Buten enthalten kann, wird einer weiteren Trennstufe 29, beispielsweise einer Butadien-Wäsche zugeführt, und dort in einen Strom 31 aus n-Butan und gegebenenfalls 1-Buten und 2-Buten und einen Strom 30 aus Butadien aufgetrennt. Der Strom 31 kann zumindest teilweise in die nicht-oxidative katalytische Dehydrierstufe 15 zurückgeführt werden.

Die Erfindung wird durch die nachfolgenden Beispiele näher erläutert.

### Beispiele

### Beispiel 1

### Herstellung eines Dehydrierkatalysatorvorläufers

1000 g eines gesplitteten ZrO₂/SiO₂-Mischoxids mit einem-ZrO₂/SiO₂-Gewichtsverhältnis von 95 : 5 der Firma Norton (USA) werden mit einer Lösung von 11,993 g SnCl₂ · 2H₂O und 7,886 g H₂PtCl₆ · 6H₂O in 600 ml Ethanol übergossen.

Das Mischoxid weist folgende Spezifikationen auf:
Siebfraktion 1,6 bis 2 mm; BET-Oberfläche: 86 m²/g; Porenvolumen: 0,28 ml/g (aus Quecksilberporosimetrie-Messung).

Das überstehende Ethanol wird am Rotationsverdampfer im Wasserstrahlpumpenvakuum (20 mbar) abgezogen. Anschließend wird jeweils unter stehender Luft zunächst 15 h bei 100 °C getrocknet und anschließend bei 560 °C während 3 h calciniert. Danach wird der getrocknete Feststoff mit einer Lösung von 7,71 g CsNO₃, 13,559 g KNO₃ und 98,33 g La(NO₃)₃· 6H₂O in 2500 ml H₂O übergossen. Das überstehende Wasser wird am Rotationsverdampfer im Wasserstrahlpumpenvakuum (20 mbar) abgezogen. Anschließend wird jeweils unter stehender Luft zunächst 15 h bei 100 °C getrocknet und anschließend bei 560 °C während 3 h calciniert.

Der dabei resultierende Katalysatorvorläufer weist eine Zusammensetzung von Pt_{0,3}Sn_{0,6}Cs_{0,5}K_{0,5}La_{3,0} (Indices repräsentieren Gewichtsverhältnisse) auf (ZrO₂)₉₅ (SiO₂)₅ als Träger (Indices repräsentieren Gewichtsverhältnisse) auf.

### Beispiel 2

### Beschickung eines Dehydrierzone A-Reaktors und Aktivierung des Katalysatorvorläufers

Mit 20 ml des erhaltenen Katalysatorvorläufers aus Beispiel 1 wird ein vertikal stehender Rohrreaktor beschickt (Reaktorlänge: 800 mm; Wanddicke: 2 mm; Innendurchmesser: 20 mm; Reaktormaterial: innenalonisiertes, d.h. mit Aluminiumoxid beschichtetes Stahlrohr; Beheizung: elektrisch mittels eines Ofens der Fa. HTM Reetz, LOBA 1100-28-650-2 auf einer längsmittigen Länge von 650 mm). Die Länge der Katalysatorschüttung beträgt 75 mm. Die Katalysatorschüttung ist auf der Längsmitte des Rohrreaktors angeordnet. Das Reaktorrestvolumen nach oben und unten ist mit Steatitkugeln als Inertmaterial (Durchmesser 4 - 5 mm), welche nach unten auf einem Kontaktstuhl aufsitzen, befüllt.

Anschließend wird das Reaktionsrohr bei einer Außenwandtemperatur längs der Heizzone von 500 °C mit 9,3 Nl/h Wasserstoff während 30 min beschickt. Anschließend wird der Wasserstoffstrom bei gleichbleibender Wandtemperatur zunächst während 30 min durch einen 23,6 Nl/h betragenden Strom aus 80 Vol.-% Stickstoff und 20 Vol.-% Luft und danach während 30 min durch einen identischen Strom aus reiner Luft ersetzt. Unter Beibehaltung der Wandtemperatur wird dann mit einem identischen Strom von N₂ während 15 min gespült und abschließend nochmals während 30 min mit 9,3 Nl/h Wasserstoff reduziert. Damit ist die Aktivierung des Katalysatorvorläufers abgeschlossen.

### Beispiel 3

### Herstellung eines Oxidehydrierkatalysators

In einem beheizbaren 10 1-Rührbehälter aus Glas werden 1750,9 g wässrige Kobaltnitrat-Lösung mit einem freien HNO₃-Gehalt von 0,2 Gew.% und einem Co-Gehalt von 12,5 Gew.-% (= 3,71 Mol Co) vorgelegt. In der vorgelegten Kobaltnitrat-Lösung werden unter Rühren 626,25 g festes Fe(NO₃)₃ · 9H₂O mit einem Fe-Gehalt von 14,2 Gew.-% (= 1,59 Mol Fe) bei Raumtemperatur gelöst. In die erhaltene Lösung werden bei Raumtemperatur 599,5 g Bismutnitrat-Lösung mit einem freien HNO₃-Gehalt von 3 Gew.-% und einem Bi-Gehalt von 11,1 Gew.-% (= 0,32 Mol Bi) zugegeben. Anschließend werden 106,23 g festes Cr(NO₃)₃ · 9 H₂O (= 0,27 Mol Cr) zugegeben. Nach dem Aufheizen auf 60 °C und weiterem Rühren wird eine rote Lösung (Lösung A) erhalten.

In einem beheizbaren 3 1-Rührbehälter aus Glas werden 2000 ml Wasser vorgelegt. Anschließend werden 2,38 g KOH (= 0,042 Mol K) und 1124,86 g (NH₄)₆Mo₇O₂₄ ·4 H₂O (= 6,37 Mol Mo) zugegeben und bei 60 °C gelöst. Die erhaltene Lösung zeigt eine leichte Trübung (Lösung B).

Anschließend wird Lösung B in Lösung A gepumpt, wobei letztere gerührt wird. In die erhaltene, 60 °C heiße, dunkelgelbe Suspension werden 102,05 g SiO₂-Sol mit einem SiO₂-Gehalt von 50 Gew.-% ("Ludox TM" der Fa. DuPont = 0,85 Mol Si) zugegeben.

Die erhaltene Suspension wird bei 60 °C 30 Minuten lang nachgerührt und anschließend sprühgetrocknet (Eingangstemperatur 370 °C, Ausgangstemperatur 110 bis 112 °C). Das erhaltene Sprühpulver wird mit 4 Gew.-% Graphit versetzt und anschließend zu Volltabletten mit einem Durchmesser von 5 mm und einer Höhe von 3 mm tablettiert. Die Volltabletten werden 6 Stunden lang bei 480 °C in einem Muffelofen auf einem mit Luft durchströmten Drahtsieb (Maschenweite 3,5 mm) und einer durchströmenden Luftmenge von 100 l/h getempert. Die kalzinierten Tabletten werden über ein Drahtsieb zu einem Katalysatorsplitt mit einem durchschnittlichen Granulatdurchmesser von 2 bis 3 mm zerkleinert.

Der Oxidehydrierkatalysator hat die nominale Zusammensetzung Mo₁₂Bi_{0,6}Fe₃Co₇Cr_{0,5}Si_{1,6}K_{0,08}Oₓ (Indices repräsentieren Atomverhältnisse).

### Beispiel 4

### Beschickung eines Dehydrierzone B-Reaktors

Mit 95 ml des erhaltenen Katalysatorvorläufers aus Beispiel 3 wird ein vertikal stehender Rohrreaktor beschickt (Reaktorlänge: 100 cm; Wanddicke: 2 mm; Innendurchmesser: 13 mm, Reaktormaterial: innenalonisiertes Stahlrohr mit darin befindlicher Thermohülse mit einem Außendurchmesser von 2 mm, die ein verschiebbares Thermoelement enthält; Beheizung: elektrisch mit 3 unterschiedlichen Heizzonen über die Reaktorlänge von 100 cm mittels Heizmanschetten der Fa. Winkler, Heidelberg, wobei eine maximale isotherme Strecke von 82 cm über den mittleren Bereich des Reaktors erreicht wird). Die Länge der Katalysatorschüttung beträgt 82 cm. Die Katalysatorschüttung befindet sich im isothermen Bereich des Rohrreaktors. Das Reaktorrestvolumen ist nach oben und unten mit Steatitkugeln als Inertmaterial (Durchmesser 2 - 3 mm) beschickt, wobei die gesamte Reaktionsrohrbeschickung nach unten auf einem Kontaktstuhl der Höhe 5 cm aufsitzt.

### Beispiel 5

### Dehydrierung von n-Butan in dem Dehydrierzone A-Reaktor

Der Dehydrierzone A-Reaktor aus Beispiel 2 wird bei einer Außenwandtemperatur längs der Heizzone von 500 °C mit einem Gemisch aus 20 Nl/h n-Butan 3,5 Nl/h Luft, 1,4 Nl/h Wasserstoff und 10 Nl/h Wasserdampf als Reaktionsgasgemisch beschickt.

n-Butan, Luft und Wasserstoff werden dabei mittels eines Massendurchflußreglers der Fa. Brooks zudosiert, während das Wasser mittels einer HPLC Pump 420 der Fa. Kontron zunächst flüssig in einen Verdampfer dosiert, in selbigem verdampft und dann mit dem n-Butan und der Luft vermischt wird. Die Temperatur des Beschickungsgasgemisches beträgt bei der Beschickung 150 °C. Mittels einer sich am Reaktorausgang befindlichen Druckregelung der Fa. REKO wird der Ausgangsdruck am Rohrreaktor auf 1,5 bar eingestellt.

Eine analytische Menge des erhaltenen Produktgasgemischs A wird auf Normaldruck entspannt und abgekühlt, wobei der enthaltene Wasserdampf auskondensiert. Das verbleibende Gas wird mittels GC (HP 6890 mit Chem.-Station, Detektoren: FID; WLD; Trennsäulen: Al₂O₃/KCI (Chrompack), Carboxen 1010 (Supelco)) analysiert. In entsprechender Weise wird auch das Beschickungsgasgemisch analysiert.

Es werden nach drei Tagen Betriebsdauer die in Tabelle 1 wiedergegebenen Analysenergebnisse erhalten:

**Tabelle 1**

| | Beschickungsgasgemisch [Vol.-%] | Produktgasgemisch [Vol.-%] |
|---|---|---|
| Methan | | 0,07 |
| Ethan | | 0,05 |
| Ethen | | < 0,01 |
| Propan | | 0,10 |
| Propen | | 0,05 |
| H₂ | 4.0 | 16,3 |
| O₂ | 2,0 | < 0,01 |
| N₂ | 8,0 | 6,8 |
| CO | | 0,03 |
| CO₂ | | 0,28 |
| Isobutan | | 0,11 |
| n-Butan | 57,4 | 33,2 |
| trans-Buten | | 5,7 |
| cis-Buten | | 4,8 |
| Isobuten | | 0,08 |
| 1-Buten | | 4,1 |
| Butadien | | 0,52 |
| H₂O | 28,6 | 27,7 |

Diesen Werten entspricht ein auf einmaligen Durchsatz bezogener n-Butanumsatz von 32 mol.-% und eine Selektivität der n-Butenbildung von 94 mol.-%. Die Selektivität der Butadienbildung entspricht 3,3 %.

### Beispiel 6

### Dehydrierung von n-Butan in dem Dehydrierzone A-Reaktor und nachfolgende Oxidehydrierung in dem Dehydrierzone B-Reaktor

Der Dehydrierzone B-Reaktor aus Beispiel 4 wird auf eine Temperatur aufgeheizt, bei der der n-Buten-Umsatz bei einmaligem Durchgang von Reaktionsgasgemisch > 99 mol-% beträgt, wobei die Innentemperatur des Reaktors mittels des in der innenliegenden Thermohülse befindlichen Thermoelementes kontrolliert wird.

Die Beschickung besteht aus einem Gemisch von 150 Nl/h Luft ( = 20 °C) und den 34,4 Nl/h des Produktgasgemisches A aus Beispiel 5 ( = 500 °C). Die Luft wird mittels eines Massendurchflußreglers der Fa. Brooks zudosiert. Die Temperatur des Beschickungsgasgemisches wird auf die Reaktoraußenwand-Temperatur gebracht. Mittels einer sich am Reaktionsrohrausgang befindlichen Druckregelung wird der Ausgangsdruck des Reaktors auf 1,3 bar eingestellt.

Hinter der Druckregelung wird das erhaltene Produktgasgemisch B (Temperatur = 330 °C) auf Normaldruck entspannt und mittels GC (HP 6890 mit Chem-Station; Detektoren: WLD; FID; Trennsäulen: Poraplot Q (Chrompack), Carboxen 1010 (Supelco)) analysiert. In identischer Weise wird auch das Beschickungsgasgemisch analysiert.

Es werden nach 3 Tagen Betriebsdauer die in Tabelle 2 wiedergegebenen Ergebnisse erhalten.

**Tabelle 2**

| | Beschickungsgasgemisch [Vol.-%] | Produktgasgemisch [Vol.-%] |
|---|---|---|
| Methan | 0,02 | 0,01 |
| Ethan | 0,01 | 0,01 |
| Ethen | < 0,01 | < 0,01 |
| Propan | 0,02 | 0,02 |
| Propen | 0,01 | < 0,01 |
| H₂ | 3,5 | 3,5 |
| O₂ | 15,7 | 11,1 |
| N₂ | 64,3 | 63,5 |
| CO | 0,01 | 1,3 |
| CO₂ | 0,06 | 1,3 |
| Isobutan | 0,02 | 0,02 |
| n-Butan | 7,1 | 7,0 |
| trans-Buten | 1,2 | < 0,01 |
| cis-Buten | 1,0 | < 0,01 |
| Isobuten | 0,02 | < 0,01 |
| 1-Buten | 0,9 | < 0,01 |
| Butadien | 0,11 | 2,6 |
| H₂O | 6,0 | 9,6 |

Diesen Werten entspricht ein auf einmaligen Durchgang bezogener n-Buten-Umsatz von 99 mol.-% und eine Selektivität der Butadienbildung von 80 mol.-%.

Die Gesamtausbeute an Butadien über beide Dehydrierzonen A und B, bezogen auf eingesetztes n-Butan, beträgt 25 %.

### 4. Vergleichsbeispiel

Das in Beispiel 5 beschriebene Beschickungsgasgemisch wird direkt in den Dehydrierzone B-Reaktor geleitet. Bei identischen Reaktionsbedingungen erfolgt keine Umsetzung des n-Butans zu Butenen oder Butadien.

## Patentansprüche

1. Verfahren zur Herstellung von Butadien aus n-Butan mit den Schritten
(A) Bereitstellung eines n-Butan enthaltenden Einsatzgastroms,
(B) Einspeisung des n-Butan enthaltenden Einsatzgasstroms in eine erste Dehydrierzone und nicht-oxidative katalytische Dehydrierung von n-Butan zu 1-Buten, 2-Buten und gegebenenfalls Butadien, wobei ein n-Butan, 1-Buten, 2-Buten, gegebenenfalls Butadien und gegebenenfalls Nebenbestandteile enthaltender erster Produktgasstrom erhalten wird,
(C) Einspeisung des n-Butan, 1-Buten, 2-Buten, gegebenenfalls Butadien und gegebenenfalls Nebenbestandteile enthaltenden ersten Produktgasstroms in eine zweite Dehydrierzone und oxidative Dehydrierung von 1-Buten und 2-Buten zu Butadien, wobei ein Butadien, n-Butan, Wasserdampf und gegebenenfalls Nebenbestandteile enthaltender zweiter Produktgasstrom erhalten wird,
(D) Gewinnung von Butadien aus dem zweiten Produktgastrom.

2. Verfahren nach Anspruch 1, **dadurch gekennzeichnet, dass** die Bereitstellung des n-Butan enthaltenden Einsatzgasstromes die Schritte umfasst
(A1) Bereitstellung eines liquefied petroleum gas (LPG)-Stroms,
(A2) Abtrennung von Propan und gegebenenfalls Methan, Ethan und Pentanen aus dem LPG-Strom, wobei ein Butane enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

3. Verfahren nach Anspruch 1 oder 2, **dadurch gekennzeichnet, dass** die nicht-oxidative katalytische Dehydrierung (B) von n-Butan als autotherme katalytische Dehydrierung durchgeführt wird.

4. Verfahren nach einem der Ansprüche 1 bis 3, **dadurch gekennzeichnet, dass** die oxidative Dehydrierung (C) mehrstufig durchgeführt wird.

5. Verfahren nach einem der Ansprüche 1 bis 4, **dadurch gekennzeichnet, dass** die Gewinnung (D) von Butadien aus dem zweiten Produktgasstrom die Schritte umfasst:
(D1) Abkühlung des Produktgasstroms mit Wasser, wobei Wasserdampf und gegebenenfalls hochsiedende organische Nebenbestandteile auskondensiert werden;
(D2) Abtrennung der in dem zweiten Produktgasstrom enthaltenen leichtsiedenden Nebenbestandteile, ausgewählt aus der Gruppe bestehend aus Wasserstoff, Kohlenmonoxid, Kohlendioxid, Stickstoff, Methan, Ethan, Ethen, Propan und Propen, wobei ein Butadien, n-Butan, gegebenenfalls 1-Buten und 2-Buten und gegebenenfalls Oxygenate als weitere Nebenbestandteile enthaltender Strom erhalten wird;
(D3) gegebenenfalls Abtrennung der Oxygenate, wobei ein Butadien, n-Butan und gegebenenfalls 1-Buten und 2-Buten enthaltender Strom erhalten wird;
(D4) Auftrennung des Butadien, n-Butan und gegebenenfalls 1-Buten und 2-Buten enthaltenden Stroms in einen n-Butan und gegebenenfalls 1-Buten und 2-Buten enthaltenden Strom und einen Butadien enthaltenden Strom;
(D5) gegebenenfalls Rückführung des n-Butan und gegebenenfalls 1-Buten und 2-Buten enthaltenden Stroms in die nicht-oxidative katalytische Dehydrierung (B).

## Claims

1. A process for preparing butadiene from n-butane comprising the steps of
(A) providing an n-butane-comprising feed gas stream,
(B) feeding the n-butane-comprising feed gas stream into a first dehydrogenation zone and nonoxidatively catalytically dehydrogenating n-butane to 1-butene, 2-butene and optionally butadiene to obtain a first product gas stream comprising n-butane, 1-butene and 2-butene, with or without butadiene and secondary components,
(C) feeding the first product gas stream comprising n-butane, 1-butene and 2-butene, with or without butadiene and secondary components, into a second dehydrogenation zone and oxidatively dehydrogenating 1-butene and 2-butene to butadiene to give a second product gas stream comprising butadiene, n-butane and steam, with or without secondary components,
(D) recovering butadiene from the second product gas stream.

2. The process according to claim 1, wherein the provision of the n-butane-comprising feed gas stream comprises the steps of
(A1) providing a liquefied petroleum gas (LPG) stream,
(A2) removing propane and optionally methane, ethane and pentanes from the LPG stream to obtain a butane-comprising stream,
(A3) removing isobutane from the butane-comprising stream to obtain the n-butane-comprising feed gas stream and optionally isomerizing the removed isobutane to an n-butane/isobutane mixture and recycling the n-butane/isobutane mixture into the isobutane removal.

3. The process according to claim 1 or 2, wherein the nonoxidative catalytic dehydrogenation (B) of n-butane is carried out as an autothermal catalytic dehydrogenation.

4. The process according to any of claims 1 to 3, wherein the oxidative dehydrogenation (C) is carried out in more than one stage.

5. The process according to any of claims 1 to 4, wherein the recovery (D) of butadiene from the second product gas stream comprises the steps:
(D1) cooling the product gas stream with water to condense out steam and any high-boiling organic secondary components;
(D2) removing the low-boiling secondary components present in the second product gas stream which are selected from the group consisting of hydrogen, carbon monoxide, carbon dioxide, nitrogen, methane, ethane, ethene, propane and propene, to obtain a stream comprising butadiene and n-butane, with or without 1-butene and 2-butene, and with or without oxygenates as further secondary components;
(D3) optionally removing the oxygenates to obtain a stream comprising butadiene and n-butane, with or without 1-butene and 2-butene;
(D4) separating the stream comprising butadiene and n-butane, with or without 1-butene and 2-butene, into a stream comprising n-butane, with or without 1-butene and 2-butene, and a stream comprising butadiene;
(D5) optionally recycling the stream comprising n-butane, with or without 1-butene and 2-butene, into the nonoxidative catalytic dehydrogenation (B).

## Revendications

1. Procédé de fabrication de butadiène à partir de n-butane comprenant les étapes
(A) mise à disposition d'un courant gazeux initial contenant du n-butane,
(B) introduction du courant gazeux initial contenant du n-butane dans une première zone de déshydrogénation et déshydrogénation catalytique non oxydative du n-butane en 1-butène, 2-butène et éventuellement butadiène, un premier courant gazeux de produits contenant du n-butane, du 1-butène, du 2-butène, éventuellement du butadiène et éventuellement des constituants secondaires étant obtenu,
(C) introduction du premier courant gazeux de produits contenant du n-butane, du 1-butène, du 2-butène, éventuellement du butadiène et éventuellement des constituants secondaires dans une seconde zone de déshydrogénation et déshydrogénation oxydative du 1-butène et du 2-butène en butadiène, un second courant gazeux de produits contenant du butadiène, du n-butane, de la vapeur d'eau et éventuellement des constituants secondaires étant obtenu,
(D) récupération du butadiène du second courant gazeux de produits.

2. Procédé selon la revendication 1, **caractérisé en ce que** la mise à disposition du courant gazeux initial contenant du n-butane comprend les étapes
(A1) mise à disposition d'un courant de gaz de pétrole liquéfiés (LPG),
(A2) séparation du propane et éventuellement du méthane, de l'éthane et des pentanes du courant de LPG, un courant contenant des butanes étant obtenu,
(A3) séparation de l'isobutane du courant contenant des butanes, le courant gazeux initial contenant du n-butane étant obtenu, et éventuellement isomérisation de l'isobutane séparé en un mélange n-butane/isobutane et recyclage du mélange n-butane/isobutane dans la séparation de l'isobutane.

3. Procédé selon la revendication 1 ou 2, **caractérisé en ce que** la déshydrogénation catalytique non oxydative (B) du n-butane est réalisée sous la forme d'une déshydrogénation catalytique autotherme.

4. Procédé selon l'une quelconque des revendications 1 à 3, **caractérisé en ce que** la déshydrogénation oxydative (C) est réalisée en plusieurs étapes.

5. Procédé selon l'une quelconque des revendications 1 à 4, **caractérisé en ce que** la récupération (D) du butadiène à partir du second courant gazeux de produits comprend les étapes :
(D1) refroidissement du courant gazeux de produits avec de l'eau, de la vapeur d'eau et éventuellement des constituants secondaires organiques de point d'ébullition élevé se condensant ;
(D2) séparation des constituants secondaires de point d'ébullition bas contenus dans le second courant gazeux de produits, choisis dans le groupe constitué de l'hydrogène, du monoxyde de carbone, du dioxyde de carbone, de l'azote, du méthane, de l'éthane, de l`éthène, du propane et du propène, un courant contenant du butadiène, du n-butane, éventuellement du 1-butène et du 2-butène et éventuellement des composés oxygénés en tant que constituants secondaires supplémentaires étant obtenu ;
(D3) éventuellement séparation des composés oxygénés, un courant contenant du butadiène, du n-butane et éventuellement du 1-butène et du 2-butène étant obtenu ;
(D4) séparation du courant contenant du butadiène, du n-butane et éventuellement du 1-butène et du 2-butène en un courant contenant du n-butane et éventuellement du 1-butène et du 2-butène et un courant contenant du butadiène ;
(D5) éventuellement recyclage du courant contenant du n-butane et éventuellement du 1-butène et du 2-butène dans la déshydrogénation catalytique non oxydative (B).
